# EUROPEAN PATENT APPLICATION

(11) **EP 3 108 896 A1**
(43) Date of publication of application: **28.12.2016**
(21) Application number: 15173156.9
(22) Date of filing: 22.06.2015
(51) Int. Cl.: A61K 39/385, C12N 15/62

(54) **IMMUNOGENIC SYNTHETIC OLIGOPEPTIDES**

(71) Applicant: Spinosa, Jean-Pierre, 1003 Lausanne (CH); Strelnikov, Evgeny, Moscow (RU)
(72) Inventor: SPINOSA, Jean-Pierre, 1003 Lausanne (CH)
(74) Representative: Grosfillier, Philippe

(57) **Abstract**

The present invention provides a method for producing immunogenic synthetic oligopeptides that are formed by minimal immune determinants uniquely present in antigens of interest and are useful for evoking specific, effective and non-crossreactive immune responses in immunotherapies and immunodiagnostics. The determinants are connected to each other by linkers and wherein that each linker contains at least one element that is not a human aminoacid.

The invention also provides a pharmaceutical preparation, such as a vaccine, which comprises said immunogenic synthetic oligopeptides. It also covers veterinary and agricultural preparations.

## Description

### Field of invention

The present invention relates to immunogenic synthetic oligopeptides that are useful for evoking specific and effective immune responses in immunotherapies and immunodiagnostics.

### State of the art

Researches for constructing immunogenic peptide sequences able to evoke effective immune responses have been conducted.
Different epitope qualities have been investigated, including hydrophobicity hydrophilicity, the protrusion index, flexibility, and secondary structure and conformational parameters.
However, today it is still not clear which features are good determinants for accurate epitope prediction. Such a situation results in unsatisfactory performances of existing prediction methods.

As a consequence of the unclear definition of immunogenic peptide epitopes, current immunotherapies against tumors and infectious pathogens are limited to the following approaches :
1) Based on entire protein antigen(s)
2) Non-immunogenic or low-immunogenic so that adjuvants have to be included in vaccine formulations
3) Accompanied by unwanted side effects due to adjuvants
4) Accompanied by cross-reactions effects due to the peptide sharing between the antigen(s) of interest, that is tumor-associated-antigens or TAAs and infectious pathogen antigens, and the proteins of the host.

For the same reasons as mentioned above current diagnostic immune assays are affected by a lack of specificity.

There is therefore a need to improve the existing immunogenic peptide sequences.

### Summary of the invention

The present invention covers a method for constructing immunogenic synthetic oligopeptides, in particular antigen epitopes, which shows several advantages with respect to the existing constructs.
The invention also concerns a pharmaceutical preparation, in particular a vaccine, which comprises an antigen obtained according to said method. It also concerns the veterinary and the agricultural fields.

The invention more precisely relates to a method and to products as defined in the claims.

The invention applies the concept that peptide uniqueness dictates the self-nonself discrimination, so that only peptide sequences not present in the host may be immunogenic toward the host, and provides a method for constructing immunogenic synthetic oligopeptides that are formed by minimal pentapeptide immune determinants unique to the antigen (or to multiple antigens) of interest and not present in the host proteome.
The expression "minimal immune determinants" refers to pentapeptides. An oligopeptide according to the invention may contain up to n amino acids (with n>5).

The method comprise three steps:
a) Dissecting the amino acid sequence of the antigen(s) of interest into a plurality of pentapeptides;
b) Selecting pentapeptides not shared with host proteins and unique to the antigen(s) of interest, and
c) Joining the selected pentapeptides with linkers, wherein each linker contains at least one element that is not a human aminoacid.

Any suitable linker can be used. It may for instance contain one or several of the following elements: amino-3-oxapentanoic acid, PEG1, PEG2, PEG4, beta amino acid, gamma amino acid, aminohexanoic acid or amino-3,6-dioxaoctanoic acid.

The term "linker" in the present document has to be indifferently understood as "linker" or "spacer", as commonly used in molecular biology.

The method for obtaining oligopeptide constructs formed by minimal determinants uniquely owned by the antigen of interest, hereafter referred to as "unique oligopeptide constructs", provides the possibility for developing immunogenic, specific and safe immunotherapies and immunodiagnostic tools.
Some of the advantages provided by the present invention are listed below :
- Unique oligopeptide constructs have the qualities of immunogenicity since they are "nonself" to the host immune system.
- Unique oligopeptide constructs have the qualities of specificity since they can induce immune responses targeting only and exclusively the antigen from which they derive.
- Unique oligopeptide constructs have the qualities of safety: being unique to the antigen of interest, there is no risk of crossreactions with the host proteins.
- Unique oligopeptide constructs are effective immunogens, while cancer entire antigens and entire antigens from infectious agents, are poorly immunogens and need adjuvants to evoke an immune response.
- Unique oligopeptide constructs guarantee high specificity and no crossreactivity in immunodiagnostics.
- Combination(s) of unique oligopeptide constructs offer the concrete possibility of protecting and/or vaccinating against multiple diseases, as for example, against a primary tumor antigen and metastasis associated-antigen(s), against multiple strains of an infectious pathogen, against multiple pathogens, and so forth.
- The presence of linkers between two adjacent pentapeptides induce some distance such that accessibility is maximised and sterical hindrance is minimal.
- The linkers improve aqueous solubility, when applicable, are immunogenically neutral and well tolerated by the organism.
- Linkers can be of various type, length and composition.
- Linkers are compatible with general peptide chemistry protocols and reagents such that they can be readily inserted
- Linkers of any type can be cumulated and can but do not need to be separated by non human amino acids.

For the preparation of the oligopeptides according to the invention several types of polymerization processes can be considered such aslactam bridge formation, metathesis, click-chemistry or chemiselective chemistry such as oximes. An advantageous polymerization method involves Cys residues for the preparation of oligopeptides with a mass of 80-100 KDa starting for peptides with a mass of 2,5-4 KDa.

Immunogenic pentameric sequences have the possibility, but do not need, to contain Cys one or more residue, as if this is the case one will observe limitation in their immunogenicity due to implication in the polymerization process.

In order to overcome this limitation of having Cys residues from the so called active region (immunogenic residues belonging to the pentamers) being involved in the polymerization process, one might use orthogonally protected Cys residues, in a tandemeric format such as Cys(Trt)/Cys(Acm), Cys(tBu)/Cys(MeBzl), Cys(STmp)/Cys(Mmt), other combinations and other Cys protected residues are not excluded (refer to colored C residues in scheme above). One can then have Cys residues in the immunogenic region for biological interaction and other Cys residues for the polymerization process.
Orthogonally protected Cys residues enable selective removal of the side chain protection (Trt, Acm, tBu, MeBzl,....) in a defined manner and thus proceed with selective polymerization with the Cys residues of choice, in particular ones located in the spacer regions( refer to other section for spacer description). Once polymerization accomplished, the other Cys, in particular those needed for immunogenicity (epitope region) can be de-protected and shall be accessible within the epitope without affecting the disulfide already present.

The present invention considerably increases the efficiency of the vaccinating against deadly tumors and threatening infections, even contemporaneously.
In addition, with the present invention there is a lack of vaccine crossreactivity, adverse effects, generation of anti-antibodies, antigen polymorphisms, lack of specificity, and others problems.

Specifically, the method produces unique oligopeptide constructs that may lead to:
- More efficacious immunotherapies against tumoral diseases,
- More efficacious immunotherapies against infectious diseases,
- More liable immunodiagnostics for tumoral and infectious diseases,
- Shorten the time length and the costs for clinical trials since unique oligopeptide construct are safe and exempt from collateral events,
- Allow administration of higher dosages and for longer time intervals thus providing the possibility of performing intensive immunotherapies,
- Eliminate many collateral events (seizures, developmental and neuronal disorders, autism, schizophrenia, immunodeficiency, sudden infant death syndrome, etc...) that sometimes have been associated with current preventive vaccination protocols (i.e, anti-polio, anti-HPV, DPT, MMR, and other vaccines),
- Allow repeated and multiple vaccinations to eradicate and/or to prevent tumoral and infectious diseases,
- Allow prompt and fast immuno-diagnosis at the very early stages of tumoral and infectious diseases.

The above cited list of advantages is of course not exhaustive.

### Example

The example uses a hypothetical amino acid sequence X as antigen of interest, and applies the peptide uniqueness principle to identify minimal pentapeptide determinants unique to the antigen of interest and not represented in the human proteome using the above mentioned 3 steps, namely:
1) *Amino acid sequence dissection into pentapeptides.* Hypothetical antigen X with a 226 amino acid long sequence (with amino acid reported in 1-letter code) equal to the primary sequence: 
MDDCNDEGHDDPGHDDTAHEHDAHFDTAHPMMAIEHDAMDHDAMEHDFVCHDF
WFHDVMGHDVNCNIVNEICVNFDTVNFHDDHDRECMECNIMEHDTMEPMMMCGF
HDCGDPTFHDPTGHDPWCHDPWDHDPWEHGHDLMCNILMDHDLMFHDLNHDLP
CNILQCNINIANEHDAPFHDAQFHDCAEHDCAEICCAFDTCAHDTCCCNICCDHDCC
EDTCCFHDC
 The X antigen sequence is dissected into 222 pentapeptides overlapping each other by four amino acids, i.e. : MDDCN, DDCND, DCNDE, CNDEGH, NDEGH, etc...
*2) Pentapeptide selection.* Each pentapeptide is analyzed for matching to the human proteome using PIR peptide match program
(see : pir.georgetown.edu/pirwww/search/peptide.shtml).
110 pentapeptides exclusively present in the antigen of interest X but absent in human proteins are selected. The selected pentapeptides are listed in Table 1:

**Table 1. Pentapeptides unique to antigen X (i.e. absent in human proteins). Listed in alphabetical order, and separated by semicolons**

| |
|---|
| AHDTC; AHEHD; AHFDT; AHPMM; AIEHD; AMDHD; AMEHD; ANEHD; APFHD; AQFHD; CAEHD; CAEIC; CAHDT; CCAFD; CCCNI; CCDHD; CCEDT; CEDTC; CGFHD; CHDPW; CMECN; CNICC; CNIME; DAMEH; DCNDE; DFWFH; DHDAM; DHDCC; DHDPW; DLMCN; DLPCN; DPGHD; DPWCH; DPWDH; DPWEH; DRECM; DTAHE; DTCAH; DTCCC; DTMEP; DVMGH; ECNIM; EHDAH; EHDCA; EHGHD; EICCA; EPMMM; FDTAH; FDTCA; FHDAQ; FHDCA; FHDCG; FHDPT; FVCHD; GHDDP; GHDPW; HDAHF; HDAME; HDAPF; HDCGD; HDDHD; HDFVC; HDFWF; HDPWC; HDPWD; HDTCC; HDTME; HDVMG; HGHDL; HPMMA; IANEH; IEHDA; IMEHD; LMCNI; LMDHD; LPCNI; LQCNI; MCGFH; MDDCN; MDHDA; MECNI; MEHDF; MEHDT; MEPMM; MFHDL; MMAIE; MMCGF; MMDDC; MMMCG; NCNIV; NEHDA; NIMEH; PMMAI; PMMMC; PTFHD; PTGHD; PWCHD; PWDHD; PWEHG; QCNIN; TCAHD; TCCCN; TMEPM; TVNFH; VCHDF; VMGHD; VNCNI; VNEIC; VNFHD; WCHDP |

*3) Construction of synthetic oligopeptide antigens.* The chosen pentapeptides listed in Table 1 are joined together in order to form synthetic oligopeptide antigens. The antigen length is preferably kept low (about 20 amino acids) to avoid/limit folding phenomena and the possible generation of conformational epitopic sequences not unique to X and potentially shared with human proteins.

At least one linker is positioned between two adjacent pentapeptides. The linker must contain at least one element which is not a human amino acid in order to avoid the fortuitous creation (in the area between two "non human" pentapeptides) of a pentapeptide present in human proteins.

In this example the linker preferably contains at least one of the following elements : amino-3-oxapentanoic acid, PEG1, PEG2, PEG4, beta amino acid, gamma amino acid, aminohexanoic acid, amino-3,6-dioxaoctanoic acid.

Optionally the following A and B approaches are used in order to preserve the foreignness of the oligopeptide construct to the human proteome.
A) FDTCAHDTCCCNI is a 13-mer peptide formed by 9 consecutively overlapping pentapeptides, all of which are absent in the human proteome. The 13-mer-peptide can be elongated by joining with other unique pentapeptides such as ECNIM, thus generating the 18 amino acid long FDTCAHDTCCCNI-ECNIM. In this case, the junction point (I-E) does not alter the foreignness of the 18-mer to the human proteome. Indeed, also the new generated pentapeptides (i.e., CCNIE, CNIEC, NIECN, IECNI) are not present in the human proteins thus further enhancing the immunogenic potency of the synthetic oligopeptide. In this case, the presence of cysteine residues in the oligopeptide FDTCAHDTCCCNI-ECNIM sequence provides the possibility of S-S bridges among multiple FDTCAHDTCCCNI-ECNIM units.
B) The 13-mer peptide FDTCAHDTCCCNI, must necessarily be linked to a specific (penta)peptide, for example, to the unique pentapeptide HDDHD, because of biological necessities such as polymorphisms, MHC restriction, tendency of specific sequences to mutate, need of immunologically hitting a specific biologic function, and so forth. This generates the 18 amino acid long oligopeptide FDTCAHDTCCCNI-HDDHD, the junction point of which (I-H) alters the foreignness of the 18-mer to the human proteome. Indeed, among the new generated pentapeptides (i.e., CCNIH, CNIHD, NIHDD, IHDDH), one (NIHDD) is present in the human T-cell receptor-associated transmembrane adapter 1 protein. Hitting this human protein signifies to hit the T-cell defensive function. In this case, one or more linkers/spacers are inserted at the juncture point, with these linkers/spacers chosen based on the ability to generate pentapeptide extraneous to the human proteome. As for the example sequence FDTCAHDTCCCNI-HDDHD, a PEG4 is inserted in the middle of junction point to give FDTCAHDTCCCNI-PEG4-HDDHD. The PEG4 insertion does not generate pentapeptides present in the human proteome.

The invention is not limited to the oligopeptide(s) defined in this example above. Any sequence constructed on the basis of the above exposed principles (from a to i) is included.
The described invention is not limited to enhance immunity, but can be used for any suitable medical, preventive, diagnostic application for neutralizing harmful auto reactive immune response(s) in autoimmune diseases by administration of specific neutralizing immunogenic peptides but also in organ transplantation, hypertension, autoimmune endocarditis, autoimmune diabetes, scleroderma, pemphigus vulgaris, and so forth by using the reverse mechanism in order to obtain decreased immunoreactivity and enhanced immuno-tolerance.
As mentioned previously it is not limited to the pharmaceutical field but also cover the veterinary and the agricultural fields.

The described invention is extended to all nucleotide sequences that, codon optimized or not, located under any kind of viral promoter, can produce the synthetic oligopeptides construct(s) described herein in in vitro as well in vivo cell systems.

## Claims

1. A method for constructing synthetic oligopeptides that are n-amino acid long (with n > 5), said method comprising the following steps:
a) Dissecting the amino acid sequence of the antigen(s) of interest into a plurality of pentapeptides,
b) Selecting pentapeptides unique to the antigen(s) of interest, but absent in the host proteins,
c) Connecting said pentapeptides to each other by linkers, wherein each linker contains at least one element that is not a human aminoacid.

2. Method according to claim 1 wherein said amino acid sequence is dissected in a way as to obtain pentapeptides that are each other consecutively overlapped by 4 aminoacids, i.e. *ABDCE, BCDEF, CDEFG,* etc..

3. Method according to claim 1 or 2 wherein the linkers contain one or several of the following elements: amino-3-oxapentanoic acid, PEG1, PEG2, PEG4, beta amino acid, gamma amino acid, aminohexanoic acid, amino-3,6-dioxaoctanoic acid.

4. Synthetic oligopeptide obtained according to the method as defined in anyone of the previous claims, wherein said oligopeptide forms one or several antigen epitopes.

5. Antigen epitope(s) according to claim 4 to be used as immunogen in an active and/or passive vaccines for tumor and/or infectious diseases.

6. Pharmaceutical preparation comprising the oligopeptide of claim 4 or 5.

7. Pharmaceutical preparation according to claim 6 to be used in active or passive vaccines.

8. Veterinary preparation comprising oligopeptides obtained according to the method as defined claims 1 to 3.

9. Agricultural preparation comprising oligopeptides obtained according to the method as defined claims 1 to 3.
